(19) Europäisches Patentamt
European Patent Office
Office européen des brevets

(11) Veröffentlichungsnummer: **0 363 458 B1**

(12) **EUROPÄISCHE PATENTSCHRIFT**

(45) Veröffentlichungstag der Patentschrift: **03.08.94**

(51) Int. Cl.[5]: **C07C 25/13**, C07C 63/70, C07C 43/225, C07C 25/18, C07C 47/575, C07D 213/26

(21) Anmeldenummer: **89903153.8**

(22) Anmeldetag: **27.02.89**

(86) Internationale Anmeldenummer:
**PCT/EP89/00181**

(87) Internationale Veröffentlichungsnummer:
**WO 89/08629 (21.09.89 89/23)**

(54) **2,3-DIFLUORBENZOLE.**

(30) Priorität: **10.03.88 DE 3807910**

(43) Veröffentlichungstag der Anmeldung:
**18.04.90 Patentblatt 90/16**

(45) Bekanntmachung des Hinweises auf die Patenterteilung:
**03.08.94 Patentblatt 94/31**

(84) Benannte Vertragsstaaten:
**CH DE FR GB IT LI**

(56) Entgegenhaltungen:

**CHEMICAL ABSTRACTS, 11th Collective Index, vols. 96-105, 1982-1986, American Chemical Society (US); no. 17175f, para. C24H27F2N&NUM;**

(73) Patentinhaber: **MERCK PATENT GmbH**
**Postfach,**
**Frankfurter Strasse 250**
**D-64271 Darmstadt(DE)**

(72) Erfinder: **REIFFENRATH, Volker**
**Jahnstrasse 18**
**D-6101 Rossdorf(DE)**
Erfinder: **KRAUSE, Joachim**
**Samuel Morse-Str. 14**
**D-6110 Dieburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft 1,4-disubstituierte 2,3-Difluorbenzole der Formel I

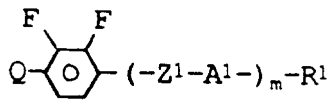

I

worin

Q

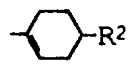

oder $R^3$

- $R^2$ H oder einen Alkyl- oder Alkenylrest mit jeweils 1 bis 15 C-Atomen, worin eine oder mehrere nicht benachbarte $CH_2$-Gruppen durch O-Atome ersetzt sein können,
- $R^3$ M, $-OR^4$, $-CO-OR^4$ oder $-O-COR^4$,
- M Li, Na oder K,
- $R^4$ H oder einen Alkylrest mit 1 bis 12 C-Atomen,
- $Z^1$ $-OCH_2-$, $-CH_2O-$, $-CH_2CH_2-$, $-CH=CH-$, $-C{\equiv}C-$ oder eine Einfachbindung,
- $A^1$ jeweils unabhängig voneinander einen
  - a) 1,4-Cyclohexylenrest, worin eine oder zwei nicht benachbarte $CH_2$-Gruppen durch O-Atome ersetzt sein können,
  - b) 1,4-Phenylenrest, worin eine oder mehrere CH-Gruppen durch N ersetzt sein können,
  - c) Rest aus der Gruppe 1,4-Cyclohexenylen, Piperin-1,4-diyl, Bicyclo(2,2,2)octylen, Naphthalin-2,6-diyl oder 1,2,3,4-Decahydronaphthalin-2,6-diyl oder 1,2,3,4-Tetrahydronaphtalin, wobei der Rest b) ein- oder zweifach durch F, Cl und/oder $-CH_3$ substituiert sein kann,
- m 0, 1, 2 oder 3,
- $R^1$ einen Alkyl-, Perfluoralkyl- oder Alkenylrest mit jeweils 1-15 C-Atomen, worin eine oder mehrere nicht benachbarte $CH_2$- bzw. $CF_2$-Gruppen durch O- und/oder S-Atome ersetzt sein können,

  $R^1$ in dem Fall

  m gleich 0 auch F, Cl oder Br

  sein kann, mit der Maßgabe, daß Q und $R^1$ nicht gleichzeitig Reste, ausgewählt aus der Gruppe F, Cl, Br, $CF_3$ und -OH sein dürfen, bedeuten.

  wobei in Verbindungen, in denen alle Reste $A^1$, ausgewählt aus der Gruppe unsubstituiertes oder ein- oder zweifach durch F substituiertes 1,4-Phenylen, bedeuten, mindestens ein $Z^1$ $-OCH_2-$, $-CH_2O-$, $-CH_2CH_2-$, $-CH=CH-$ oder $-C{\equiv}C-$ bedeutet,

  sowie deren reaktionsfähige Derivate.

Fluorierte Oligophenyle der Formel T,

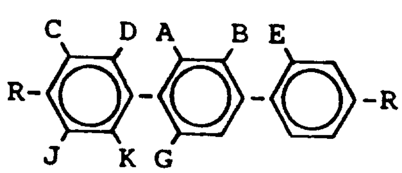

T

worin

die terminalen Substituenten

R und R' jeweils unabhängig voneinander, gegebenenfalls mit CN oder mit mindestens einem Halogenatom substituierte , Alkyl- oder Alkenylreste mit bis zu 15 C-Atomen, worin eine oder mehrere nicht benachbarte $CH_2$-Gruppen dieser Reste auch durch -O-, -S-, -CO-, -O-CO-,

-CO-O-, -O-CO-O- oder -C≡C- ersetzt sein können, einer dieser Reste R auch eine Gruppe der Formel

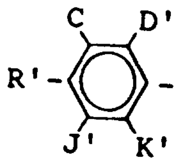

bei einem der folgenden Paare von lateralen Substituenten beide Substituenten Fluor sind: (A,B), (C,D), (C',D')

und alle übrigen lateralen Substituenten Wasserstoff oder Fluor bedeuten.

sind Gegenstand der unveröffentlichten Patentanmeldungen P 38 07 956, P 38 07 862, GB 88 06 220 und WO 87-00515.

In der JP 59-155 485 werden flüssigkristalline Mischungen beschrieben, die Biphenylcyclohexane der Formel

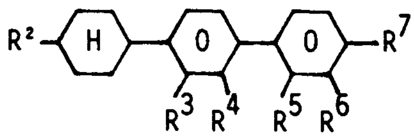

$R^{3-6}$ : H oder Halogen

$R^7$ : Alkyl, Alkoxy oder CN

$R^2$ : Alkyl

enthalten.

In der Präparativen Organischen Chemie sind Verfahren zur Metallierung von Aromaten von zunehmender Bedeutung. Es hat sich gezeigt, daß bei substituierten Aromaten aufgrund von Orientierungseffekten regioselektive Metallierungen möglich sind. So lassen sich beispielsweise 1,2- und 1,3-Difluorbenzol jeweils regiospezifisch in der ortho-Position zu 2,3- bzw. 2,6-Difluorphenyllithium metallieren.

Die lithiierten Verbindungen sind unterhalb von -50 °C stabil und stellen wertvolle Zwischenstufen bei der Synthese von vielen Difluoraromaten dar (A.M. Roe, Chem. Comm. 22, 582, 1965).

Es wurde nun überraschenderweise gefunden, daß in der 1-Position geeignet substituierte 2,3-Difluorbenzole selektiv in der 4-Position metallierbar sind. Man kann also 1,4-disubstituierte 2,3-Difluorbenzole herstellen, indem man entweder in 1-Position substituierte 2,3-Difluorbenzole mit einem metallorganischen Reagenz deprotoniert und gegebenenfalls mit einem Elektrophil umsetzt, oder, ausgehend von 1,2-Difluorbenzol, zunächst, wie oben beschrieben, in der einen ortho-Position deprotoniert, dann mit einem Elektrophil umsetzt und den Prozeß anschließend mit einem zweiten Äquivalent Base und gegebenenfalls einem Elektrophil an der zweiten ortho-Position (ortho zu F) wiederholt.

Die nach diesen Verfahren erhältlichen Verbindungen sind je nach Substitution wertvolle Zwischenstufen bei der Synthese von flüssigkristallinen Verbindungen oder Verbindungen, die als Komponenten flüssigkristalliner Phasen deren dielektrische Anisotropie oder andere Parameter verbessern. Sie sind jedoch ganz allgemein als Zwischenprodukte in der industriellen organischen Chemie von Interesse.

Gegenstand der Erfindung sind daher Verbindungen der Formel I.

Gegenstand der Erfindung ist weiterhin ein Verfahren zur Herstellung von Verbindungen der Formel I, dadurch gekennzeichnet, daß man Verbindungen, die ansonsten der Formel I entsprechen, worin aber Q H bedeutet, mit einem metallorganischen Reagens in der 4-Position deprotoniert und gegebenenfalls weiter mit einem Elektrophil, wie Halogen (gegebenenfalls mit anschließender Substitution des Halogenatoms gegen -CN), Ethylenoxid, einem Peroxid, Disulfid oder Schwefel (gegebenenfalls mit anschließender Veretherung oder Veresterung), einer reaktiven Carbonylverbindung oder Kohlendioxid (gegebenenfalls mit anschließender Veresterung oder Dehydratisierung), umsetzt.

Gegenstand der Erfindung ist schließlich die Verwendung von Verbindungen der Formel I als Zwischenprodukte zur Synthese von flüssigkristallinen Verbindungen.

Der Einfachheit halber bedeuten im folgenden A°

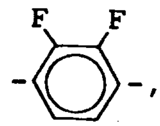

$R^2$-Che- 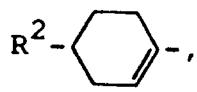

a, b und c die in Formel I für $A^1$ unter a, b und c definierten Reste, Nap Naphthalin-2,6-diyl, Pip Piperidin-1,4-diyl, Che Cyclohexenylen und BCO Bicyclo(2.2.2)octylen.

Die Verbindungen der Formel I umfassen dementsprechend Verbindungen der Teilformeln Ia bis Ih

$R^3$-A°-$R^1$ Ia

$R^2$-Che-A°-$R^1$ Ib

$R^3$-A°-$Z^1$-$A^1$-$R^1$ Ic

$R^2$-Che-A°-$Z^1$-$A^1$-$R^1$ Id

$R^3$-A°-$Z^1$-$A^1$-$Z^1$-$A^1$-$R^1$ Ie

$R^2$-Che-A°-$Z^1$-$A^1$-$Z^1$-$A^1$-$R^1$ If

$R^3$-A°-$Z^1$-$A^1$-$Z^1$-$A^1$-$Z^1$-$A^1$-$R^1$ Ig

$R^2$-Che-A°-$Z^1$-$A^1$-$Z^1$-$A^1$-$Z^1$-$A^1$-$R^1$ Ih

Darunter sind diejenigen der Teilformeln Ia bis If bevorzugt.

Die bevorzugten Verbindungen der Teilformel Ic umfassen solche der Unterformeln Ic1 bis Ic6

$R^3$-A°-$Z^1$-a-$R^1$ Ic1

$R^3$-A°-$Z^1$-b-$R^1$ Ic2

$R^3$-A°-$Z^1$-Nap-$R^1$ Ic3

$R^3$-A°-$Z^1$-Pip-$R^1$ Ic4

$R^3$-A°-$Z^1$-BCO-$R^1$ Ic5

$R^3$-A°-$Z^1$-Che-$R^1$ Ic6

Darunter sind diejenigen der Unterformeln Ic1, Ic2, Ic3, Ic5, und Ic6 besonders bevorzugt.

Die bevorzugten Verbindungen der Teilformel Id umfassen solche der Unterformeln Id1 bis Id6

$R^2$-Che-A°-$Z^1$-a-$R^1$ Id1

$R^2$-Che-A°-$Z^1$-b-$R^1$ Id2

$R^2$-Che-A°-$Z^1$-Nap-$R^1$ Id3

$R^2$-Che-A°-$Z^1$-Pip-$R^1$ Id4

$R^2$-Che-A°-$Z^1$-BCO-$R^1$ Id5

$R^2$-Che-A°-$Z^1$-Che-$R^1$ Id6

Darunter sind diejenigen der Unterformeln Id1, Id2, Id3, Id5 und Id6 besonders bevorzugt.

Die bevorzugten Verbindungen der Teilformel Ie umfassen solche der Unterformeln Ie1 bis Ie8

$R^3$-A°-$Z^1$-a-$Z^1$-a-$R^1$ Ie1

$R^3$-A°-$Z^1$-a-$Z^1$-b-$R^1$ Ie2

$R^3$-A°-$Z^1$-a-$Z^1$-c-$R^1$ Ie3

$R^3$-A°-$Z^1$-b-$Z^1$-a-$R^1$ Ie4

$R^3$-A°-$Z^1$-b-$Z^1$-b-$R^1$ Ie5

$R^3$-A°-$Z^1$-b-$Z^1$-c-$R^1$ Ie6

$R^3$-A°-$Z^1$-c-$Z^1$-a-$R^1$ Ie7

$R^3$-A°-$Z^1$-c-$Z^1$-b-$R^1$ Ie8

Darunter sind diejenigen der Unterformeln Ie1, Ie2, Ie4, Ie5, Ie7 und Ie8 besonders bevorzugt.

Die bevorzugten Verbindungen der Teilformel If umfassen solche der Unterformeln If1 bis If8

$R^2$-Che-A°-$Z^1$-a-$Z^1$-a-$R^1$ If1

$R^2$-Che-A°-$Z^1$-a-$Z^1$-b-$R^1$ If2

$R^2$-Che-A°-$Z^1$-a-$Z^1$-c-$R^1$ If3

$R^2$-Che-A°-$Z^1$-b-$Z^1$-a-$R^1$ If4

$R^2$-Che-A°-$Z^1$-b-$Z^1$-b-$R^1$ If5

$R^2$-Che-A°-$Z^1$-b-$Z^1$-c-$R^1$ If6

$R^2$-Che-A°-$Z^1$-c-$Z^1$-a-$R^1$ If7

$R^2$-Che-A°-$Z^1$-c-$Z^1$-b-$R^1$ If8

Darunter sind diejenigen der Unterformeln If1, If2, If4, If5, If7 und If8 besonders bevorzugt.

In den Verbindungen der vor- und nachstehend genannten Formeln bedeuten $R^1$ und $R^2$ jeweils unabhängig voneinander vorzugsweise einen Alkyl-, Alkenyl-, Alkoxy- oder einen Oxaalkylrest mit jeweils 1 bis 12 C-Atomen, $R^1$ auch einen Perfluoralkylrest mit 1 bis 12 C-Atomen.

Falls $R^1$ und/oder $R^2$ Alkylreste und/oder Alkoxyreste bedeuten, so können diese geradkettig oder verzweigt sein. Vorzugsweise sind die geradkettig und bedeuten demnach bevorzugt Methyl, Ethyl, Propyl, Butyl, Pentyl, Hexyl, Heptyl, Methoxy, Ethoxy, Propoxy, Butoxy, Pentoxy, Hexoxy oder Heptoxy, ferner Octyl, Nonyl, Decyl, Undecyl, Dodecyl, Octoxy, Nonoxy, Decoxy, Undecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl,

Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls $R^1$ und/oder $R^2$ Alkenylreste bedeuten, so können diese geradkettig oder verzweigt sein. Vorzugsweise sind sie geradkettig und haben 2 bis 10 C-Atome. Sie bedeuten demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Weiterhin bevorzugt sind Verbindungen, in denen m gleich 0 und $R^1$ gleich Halogen wie F, Cl oder Br bedeutet.

$R^3$ bedeutet M, $-OR^4$, $-CO-OR^4$ oder $-O-COR^4$. Insbesondere bevorzugt sind Li, K, $OR^4$ und $-CO-OR^4$.

M bedeutet vorzugsweise Li oder K.

$R^4$ bedeutet vorzugsweise H oder einen Alkylrest mit 1 bis 10 C-Atomen, insbesondere H oder einen unverzweigten Alkylrest wie Methyl, Ethyl, Propyl, Butyl, Pentyl, Hexyl, Heptyl, Octyl, Nonyl, Decyl, Undecyl oder Dodecyl.

$Z^1$ bedeutet vorzugsweise eine Einfachbindung, $-CH_2O-$, $-OCH_2-$, $-CH_2-CH_2-$ oder $-CH=CH-$, insbesondere eine Einfachbindung, $-CH_2CH_2-$, $-CH_2O-$ oder $-OCH_2-$.

a bedeutet vorzugsweise einen 1,4-Cyclohexylen- oder 1,3-Dioxan-2,5-diylrest.

b bedeutet vorzugsweise einen einfach durch F, Cl, oder $-CH_3$ substituierten 1,4-Phenylen-, Pyridin 2,5-diyl- oder Pyrimidin-2,5-diyl-, sowie die stellungsisomeren Pyridin- und Pyrimidinreste. Insbesondere bevorzugt ist ein unsubstituierter oder substituierter 1,4-Phenylenrest und ein unsubstituierter Pyridin-2,5-diyl- oder Pyrimidin-2,5-diylrest.

c bedeutet besonders bevorzugt Che, Pip, Nap und BCO, insbesondere Che.

Bevorzugt enthalten die Verbindungen der Formel I nicht mehr als einen heterocyclischen Rest.

m bedeutet vorzugsweise 0, 1 oder 2.

Insbesondere bevorzugt sind diejenigen Verbindungen der Teilformeln, in denen die Reste $R^1$, $R^2$, $R^3$, $R^4$, $Z^1$, a, b und c die angegebenen bevorzugten Bedeutungen besitzen.

Ganz besonders bevorzugte erfindungsgemäße Verbindungen der Teilformel Ia sind demnach diejenigen der Teilformeln Ia1, Ia5 und Ia7,

$M-A^{\circ}-R^1$ Ia1

$HO-A^{\circ}-R^1$ Ia5

$HO-OC-A^{\circ}-R^1$ Ia7

worin M Li oder K und $R^1$ einen Alkyl-, Alkenyl- oder Alkoxyrest mit bis zu 12 C-Atomen bedeutet.

Die Herstellung der Verbindungen der Formel I, in denen Q M bedeutet, erfolgt nach dem erfindungsgemäßen Verfahren durch Deprotonierung von entsprechenden Verbindungen der Formel I, worin Q aber H bedeutet.

Hierin sind metallorganische Reagenzien und Basensysteme, die solche Reagenzien enthalten, geeignet.

Unter den metallorganischen Reagenzien sind Alkyl- oder Arylmetallverbindungen oder Alkalimetallamide besonders geeignet. Die Basensysteme enthalten zusätzlich noch einen Aktivator, vorzugsweise Komplexbildner wie Amine oder Amide, so z. B. Hexamethylphosphorsäuretriamid (HMPT), Tetramethylethylendiamin (TMEDA) oder N,N-Dimethylpropylenharnstoff (DMPU) oder Metallaustauschreagenzien wie z. B. Kalium-tert.-butanolat (KOT).

Die Reaktion wird in inerten Lösungsmitteln oder Gemischen davon wie Ethern und/oder Kohlenwasserstoffen bei tiefen Temperaturen, im allgemeinen unterhalb von -50 °C, unter Schutzgas durchgeführt.

Durch Umsetzung der auf diese Weise entstandenen metallorganischen Verbindungen mit einem Elektrophil sind Verbindungen der Formel I mit Substituenten ungleich M in der 4-Position zugänglich. Die Reaktionsbedingungen sind für die genannten Umsetzungen bekannt und können den Standardwerken der Präparativen Organischen Chemie entnommen werden, z. B. HOUBEN-WEYL, Methoden der Organischen Chemie, Georg-Thieme-Verlag, Stuttgart oder ORGANIC SYNTHESES, J. Wiley, New York - London-Sydney. Dabei kann man auch von an sich bekannten, hier nicht näher erwähnten Varianten Gebrauch machen.

Phenole der Formel I (Q = OH) können hergestellt werden, indem man die entsprechenden metallorganischen Verbindungen (Q = M) mit Nitrobenzol oxidiert oder mit Sauerstoff (Einleiten von gasförmigen $O_2$ in die Reaktionslösung) oder einem Peroxid umsetzt. Ein bevorzugtes Peroxid ist beispielsweise Lithium-tert.-butylperoxid. Weiterhin liefert die Spaltung von Phenolethern mit Lewissäuren ebenfalls Phenole.

Carbonsäuren der Formel I (Q = COOH) können hergestellt werden, indem man entsprechende metallorganische Verbindungen (Q = M) mit Kohlendioxid umsetzt, indem man beispielsweise getrocknetes, gasförmiges $CO_2$ in die Reaktionslösung einleitet oder die Lösung mit Trockeneis versetzt. Ether der Formel I (vgl. die Definitionen für $R^1$, $R^2$, $R^3$, $Z^1$) können hergestellt werden, indem man entsprechende Hydroxybindungen verethert. Dabei wird die Hydroxyverbindung zweckmäßig zunächst in ein entsprechendes Metallderivat, z. B. durch Behandeln mit NaH, $NaNH_2$, NaOH, KOH, $Na_2CO_3$ oder $K_2CO_3$ in das entsprechende Alkalimetallalkoholat oder Alkalimetallphenolat überführt. Dieses kann dann mit dem entsprechenden Alkylhalogenid, -sulfonat oder Dialkylsulfonat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100 °C.

Ester der Formel I ($R^3$ = -CO-$OR^4$, -O-$COR^4$) können durch Veresterung entsprechender Carbonsäuren (oder ihren reaktionsfähigen Derivaten) mit Alkoholen (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1-4 C-Atomen in der Alkylgruppe.

Als reaktionsfähige Derivate der Alkohole kommen insbesondere die entsprechenden Metallalkoholate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff, Tetrachlorethylen oder Dichlormethan und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z. B. Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +180 °C. Bei diesen Temperaturen sind die Veresterungsreaktionen in der Regel nach 15 Minuten bis 48 Stunden beendet.

Besonders bevorzugt sind unter milden Bedingungen verlaufende Veresterungen. Dies kann durch Reaktion mit geeigneten wasserentziehenden Mitteln wie vorzugsweise Dicyclohexylcarbodiimid in einem angegebenen inerten Lösungsmittel erreicht werden.

Im einzelnen hängen die Reaktionsbedingungen für die Veresterung weitgehend von der Natur der verwendeten Ausgangsstoffe ab. So wird eine freie Carbonsäure mit einem freien Alkohol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol zunächst in das Natrium- oder Kaliumalkoholat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20 °C.

Verbindungen der Formel I mit Q = $R^2$-Che- können durch Reaktion der entsprechenden Verbindungen mit Q = M mit 4-substituierten Cyclohexanonen und anschließender Dehydratisierung synthetisiert werden.

Auf der anderen Seite sind Verbindungen der Formel I herstellbar, indem man 2,3-Difluorbenzolderivate die in 1-Stellung durch Q substituiert sind, in der 4-Stellung deprotoniert und gegebenenfalls mit einem Elektrophil weiter umsetzt. Dafür sind wegen möglicher Konkurrenzreaktionen, z. B. Addition an die Carbonylgruppe, nur wenige der für Q definierten Substituenten geeignet. Besonders geeignet für Q sind F, $OR^4$ ($R^4 \neq H$) und $R^2$-Che.

Es können dann auch andere Elektrophile, wie im folgenden beschrieben, eingesetzt werden.

Zur Herstellung von alkylverknüpften Verbindungen der Formel I (z. B. $Z^1$ = $-CH_2-CH_2-$ oder m = O und $R^1$ = Alkyl oder Oxaalkyl) können die metallorganischen Verbindungen mit entsprechenden Alkylhalogeniden, vorzugsweise Bromiden oder Iodiden umgesetzt werden. Ein anderer Syntheseweg ist beispielsweise die Umsetzung der metallorganischen Verbindungen mit Aldehyden, nachfolgender Dehydratisierung und abschließender Reduktion der entstandenen Doppelbindung.

Verbindungen der Formel I, in denen $Z^1$ $-CH=CH-$ oder $-C{\equiv}C-$bedeutet, können auf dem oben beschriebenen Weg synthetisiert werden: Keine Reduktion der Doppelbindung bzw. Oxidation der Doppelbindung zur Dreifachbindung durch Addition von Brom und Eliminierung von zwei Äquivalenten Bromwasserstoff.

Verbindungen der Formel I, in denen ein aromatisches System direkt mit dem Difluorphenylenrest verknüpft ist, können hergestellt werden, indem die metallorganischen Verbindungen zunächst durch einen Metall-Metall-Austausch in andere, vorzugsweise übergangsmetallorganische Verbindungen überführt werden und anschließend unter katalytischen Bedingungen mit einem Aryl- oder Heteroarylhalogenid umgesetzt werden. Die transmetallierten Verbindungen sind aber auch aus den entsprechenden Halogeniden, beispielsweise Iodiden, durch Metall-Halogen-Austausch zugänglich. Bevorzugte Übergangsmetalle sind Ti, Zn, Cu, Zr, Ni und Pd, insbesondere Ti und Zn. Außer den Übergangsmetallen sind auch Mg, Al und Si bevorzugt. Es können aber auch andere Verbindungen der Formel I, deren Synthese weiter oben beschrieben ist, zur Erhöhung der Reaktionsselektivität aus den entsprechenden transmetallierten Verbindungen hergestellt werden.

Beispielsweise werden zur Umsetzung mit aromatischen Halogenverbindungen die Lithium-2,3-difluorphenylverbindungen in Zink- (vgl. DE OS 36 32 410) oder Titanverbindungen (vgl. DE OS 37 36 489) überführt.

Aus diesen transmetallierten Verbindungen können dann beispielsweise Ketone der Formel I durch Umsetzung mit einem Carbonsäurechlorid unter Übergangsmetallkatalyse hergestellt werden.

Eine weitere wichtige Substanzklasse sind die 2,3-Difluorphenylboronsäuren und deren Ester. Sie können in Analogie zu A.M. Roe, Chem. Comm. 22, 582 (1965) dargestellt und zum Beispiel nach dort zitierter Literatur in Phenolderivate überführt werden.

Verbindungen der Formel I, in denen ein 1,4-Phenylenrest oder ein 1,4-Cyclohexylenrest direkt mit dem Difluorphenylenrest verknüpft ist, können auch hergestellt werden, indem man die entsprechenden metallorganischen Verbindungen zunächst mit Cyclohexanon oder entsprechenden Derivaten davon umsetzt, anschließend dehydratisiert ($Z^1$ = Einfachbindung, $A^1$ = Cyclohexenylen) und abschließend oxidiert bzw. reduziert.

Dioxanderivate der Formel I ($A^1$ bedeutet 1,4-Cyclohexylen, worin zwei $CH_2$-Gruppen durch O-Atome ersetzt sind) werden zweckmäßig durch Reaktion eines entsprechenden Aldehyds mit einem entsprechenden 1,3-Diol (oder einem seiner reaktionsfähigen Derivate) hergestellt, vorzugsweise in Gegenwart eines inerten Lösungsmittels wie Benzol oder Toluol und/oder eines Katalysators z. B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangstoffe eignen sich in erster Linie Acetale. Erfindungsgemäße Verbindungen, in denen ein Pyridinring direkt mit dem 2,3-Difluorphenylenrest verknüpft ist, können beispielsweise durch Kopplung von 2-Brompyridinderivaten mit einer 2,3-Difluorphenylmetallverbindung, bsw. Titan, unter Übergangsmetallkatalyse hergestellt werden.

Analoge Pyrimidinverbindungen sind herstellbar aus 1-Cyano-2,3-difluoryhenylenverbindungen durch Überführung in einen Imidoester und Reaktion mit einem entsprechenden Dialdehydacetal in Gegenwart von Ammoniak.

Die Ausgangsverbindungen zur Darstellung von Verbindungen der Formel I sind teilweise bekannt, so z. B. 1,2-Difluor-3-isopropylbenzol (M. Attina et al., Tetrahedron Lett. 23, 3525) und 2,3-Difluorbenzylalkohol (EP 10879), teilweise neu. Die Neuen unter ihnen sind Verbindungen der Formel II,

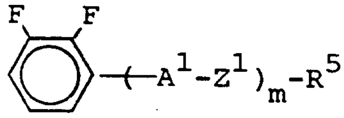

II

worin

| | |
|---|---|
| $A^1$, $Z^1$ und m | den Definitionen gemäß Formel I entsprechen und |
| $R^5$ | im Falle m ungleich 0 |
| | H oder einen Alkyl-, Perfluoralkyl oder Alkenylrest mit jeweils 1 bis 15 C-Atomen bedeutet, wobei in diesen Resten eine oder mehrere nicht benachbarte $CH_2$- bzw. $CF_2$- |

Gruppen durch O- und/oder S-Atome ersetzt sein können, ausgenommen H für den Fall, daß $-(-A^1-Z^1-)_{\overline{m}}$ einen unsubstituierten oder einen einfach durch F substituierten 1,4-Phenylenrest bedeutet,

im Falle m gleich 0

einen unverzweigten Alkylrest mit 2 bis 15 C-Atomen oder einen Perfluor- oder Alkenylrest mit 2 bis 15 C-Atomen, wobei in diesen Resten eine oder mehrere nicht benachbarte $CH_2$- bzw. $CF_2$-Gruppen durch O- und/oder S-Atome ersetzt sein können.

Sie sind ebenfalls Gegenstand der Erfindung und können ihrerseits hergestellt werden, indem man 2,3-Difluorbenzol, wie oben beschrieben, metalliert und mit einem entsprechenden Elektrophil umsetzt.

Hierbei können in wechselnden Mengen nebenbei auch die 1,4-dimetallierten 2,3-Difluorbenzole entstehen. Umsetzung mit einem Elekrophil ergibt dann daraus die entsprechenden symmetrischen 1,4-disubstituierten 2,3-Difluorbenzole.

Bevorzugte, erfindungsgemäße Verbindungen der Formel II sind

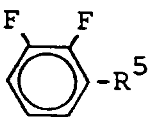 , 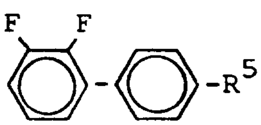 und 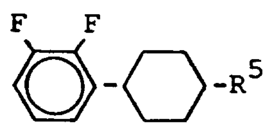

Die folgenden Beispiele sollen die Erfindung näher erläutern, ohne sie zu begrenzen.

Übliche Aufarbeitung bedeutet: Ansäuern mit verdünnter Salzsäure, Abtrennen der organischen Phase, Extraktion mit Diethylether, Dichlormethan oder Toluol, Trocknen der organischen Phase, Eindampfen und Reinigen durch Chromatographie, Umkristallisation und/oder Destillation.

Folgende Abkürzungen werden verwendet:

| | |
|---|---|
| n-BuLi | n-Butyllithium |
| t-BuOOH | tert. Butylperoxid |
| DDQ | Dichlordicyanobenzochinon |
| DMPU | N,N-Dimethylpropylenharnstoff |
| Fp | Festpunkt |
| I | Isotrop |
| K | Kristallin |
| KOT | Kalium-tert.-butoxid |
| Kp | Kochpunkt |
| LiOOT | Lithium-tert.-butylperoxid |
| N | Nematisch |
| S | Smektisch |
| THF | Tetrahydrofuran |
| TMEDA | Tetramethylendiamin |
| pTsOH | p-Toluolsulfonsäure |

Beispiel 1

Metallierung von Difluorbenzolderivaten

a) Zu einer auf -70 °C gekühlten Lösung von 0,1 mol Difluorbenzolderivat in 200 ml THF wird langsam 0,1 mol n-BuLi (1,5 M in Hexan) zugetropft und noch 6 Stunden bei dieser Temperatur weitergerührt.

b) In Abänderung zur Vorschrift 1a) wird die Lösung des Difluorbenzolderivates mit 0,1 mol TMEDA versetzt und 1 Stunde bei -70 °C weitergerührt.

c) Zu einer auf -90° bis -100 °C gekühlten Lösung von 0,1 mol des Difluorbenzolderivates und 0,1 mol KOT in 200 ml THF wird 0,1 mol n-BuLi (1,5 M in Hexan) getropft und noch 5 Minuten bei dieser Temperatur weitergerührt.

Beispiel 2

Darstellung von 3-Pentyl-1,2-difluorbenzol

Zu der nach 1c) hergestellten Lösung von 1,2-Difluorphenylkalium in THF wird 0,1 mol Pentylbromid und anschließend 13 ml DMPU zugetropft Nach 60minütigem Rühren unterhalb -85 °C wird innerhalb 30 Minuten auf -40 °C erwärmt und bei dieser Temperatur mit Wasser versetzt. Anschließend wird wie üblich aufgearbeitet. $Kp_{30}$: 112 °C

Analog werden hergestellt:
3-Ethyl-1,2-difluorbenzol
3-Propyl-1,2-difluorbenzol, $Kp_{30}$: 72 °C
3-Butyl-1,2-difluorbenzol
3-Hexyl-1,2-difluorbenzol
3-Heptyl-1,2-difluorbenzol
3-Octyl-1,2-difluorbenzol, $Kp_{25}$: 145°
3-[2-(Cyclohexyl)-ethyl]-1,2-difluorbenzol
3-[2-(trans-4-Pentylcyclohexyl)-ethyl]-1,2-difluorbenzol, $Kp_{0,5}$: 135 °C.

Beispiel 3

Darstellung von 4-Propyl-2,3-difluorbenzoesäure

3-Propyl-1,2-difluorbenzol (erhältlich nach Beispiel 2) wird, wie in 1c) beschrieben, metalliert. Bei -70 °C wird langsam 20 g Trockeneis zugegeben. Anschließend wird auf -20 °C erwärmt. Nach üblichem Aufarbeiten erhält man farblose Kristalle, Fp. 148 °C.

Analog werden hergestellt:
4-Methyl-2,3-difluorbenzoesäure
4-Butyl-2,3-difluorbenzoesäure
4-Pentyl-2,3-difluorbenzoesäure
4-Heptyl-2,3-difluorbenzoesäure
4-Methoxy-2,3-difluorbenzoesäure
4-Ethoxy-2,3-difluorbenzoesäure
4-Butoxy-2,3-difluorbenzoesäure
4-Pentoxy-2,3-difluorbenzoesäure
4-Heptoxy-2,3-difluorbenzoesäure
4-Octoxy-2,3-difluorbenzoesäure, Fp: 105 °C
4-(4-Methylcyclohexenyl)-2,3-difluorbenzoesäure
4-(4-Ethylcyclohexenyl)-2,3-difluorbenzoesäure
4-(4-Propylcyclohexenyl)-2,3-difluorbenzoesäure
4-(4-Pentylcyclohexenyl)-2,3-difluorbenzoesäure
4-(4-Heptylcyclohexenyl)-2,3-difluorbenzoesäure
4-(4-Methylphenyl)-2,3-difluorbenzoesäure
4-(4-Ethylphenyl)-2,3-difluorbenzoesäure
4-(4-Propylphenyl)-2,3-difluorbenzoesäure
4-(4-Pentylphenyl)-2,3-difluorbenzoesäure
4-(4-Heptylphenyl)-2,3-difluorbenzoesäure
4-(4-Methoxyphenyl)-2,3-difluorbenzoesäure
4-(4-Propoxyphenyl)-2,3-difluorbenzoesäure
4-(4-Pentoxyphenyl)-2,3-difluorbenzoesäure.

Beispiel 4

Darstellung von 4-Ethoxy-2,3-difluorphenol

2,3-Difluor-ethoxybenzol (erhältlich durch Alkylierung von 2,3-Difluorphenol mit Kaliumcarbonat/Ethyliodid) wird nach 1b) metalliert. Bei dieser Temperatur wird 0,12 mol LiOOT (Lösung in Hexan/Diethylether, hergestellt durch Zutropfen von 0,12 mol n-BuLi (1,5 M in Hexan), in eine Lösung von 0,12 mol t-BuOOH in 100 ml Diethylether bei -5° bis 0 °C) bei -60° bis -65 °C hinzugetropft. Nach

30minütigem Rühren bei -60 °C wird innerhalb 2 Stunden auf Raumtemperatur erwärmt. Nach üblichem Aufarbeiten (Umkristallisation aus Petrolether) erhält man farblose Kristalle, Fp. 73 °C.

Analog werden hergestellt:

4-Methyl-2,3-difluorphenol
4-Ethyl-2,3-difluorphenol
4-Propyl-2,3-difluorphenol
4-Butyl-2,3-difluorphenol
4-Pentyl-2,3-difluorphenol, $Kp_{0.5}$: 77 °C
4-Hexyl-2,3-difluorphenol
4-Octyl-2,3-difluorphenol
4-Nonyl-2,3-difluorphenol
4-Decyl-2,3-difluorphenol
4-Heptyl-2,3-difluorphenol
4-Methoxy-2,3-difluorphenol
4-Propoxy-2,3-difluorphenol
4-Butoxy-2,3-difluorphenol
4-Pentoxy-2,3-difluorphenol
4-Hexoxy-2,3-difluorphenol
4-Heptoxy-2,3-difluorphenol
4-Octoxy-2,3-difluorphenol
4-Nonoxy-2,3-difluorphenol
4-Decoxy-2,3-difluorphenol
4-(4-Methylcyclohexenyl)-2,3-difluorphenol
4-(4-Ethylcyclohexenyl)-2,3-difluorphenol
4-(4-Propylcyclohexenyl)-2,3-difluorphenol
4-(4-Pentylcyclohexenyl)-2,3-difluorphenol
4-(4-Heptylcyclohexenyl)-2,3-difluorphenol
4-(4-Methylphenyl)-2,3-difluorphenol
4-(4-Ethylphenyl)-2,3-difluorphenol
4-(4-Propylphenyl)-2,3-difluorphenol
4-(4-Pentylphenyl)-2,3-difluorphenol
4-(4-Heptylphenyl)-2,3-difluorphenol
4-(4-Methoxyphenyl)-2,3-difluorphenol
4-(4-Propoxyphenyl)-2,3-difluorphenol
4-(4-Pentoxyphenyl)-2,3-difluorphenol
4-[3-(2-(trans-4-pentylcyclohexyl)-ethyl]-2,3-difluorphenol, Fp: 61 °C.

Beispiel 5

Darstellung von 3-[4-(4-Propylphenyl)-cyclohexenyl]-1,2-difluorbenzol

Zu der nach 1a) hergestellten Lösung von 1,2-Difluorphenyllithium in THF/Hexan wird bei -70 °C eine Lösung von 0,1 mol 4-(4-Propylphenyl)-cyclohexanon in 50 ml THF getropft. Danach wird unter Rühren innerhalb 2 Stunden auf Raumtemperatur erwärmt. Nach üblichem Aufarbeiten wird der Rückstand in 150 ml Toluol nach Zugabe von 1 g p-TsOH 4 Stunden am Wasserabscheider zum Sieden erhitzt. Nach dem Abkühlen wird wie üblich aufgearbeitet (Umkristallisation aus 150 ml Methanol/Ethanol 1:1).

Analog werden hergestellt:

3-[4-(4-Methylphenyl)-cyclohexenyl]-1,2-difluorbenzol
3-[4-(4-Ethylphenyl)-cyclohexenyl]-1,2-difluorbenzol
3-[4-(4-Pentylphenyl)-cyclohexenyl]-1,2-difluorbenzol
3-[4-(4-Hexylphenyl)-cyclohexenyl]-1,2-difluorbenzol
3-[4-(4-Heptylphenyl)-cyclohexenyl]-1,2-difluorbenzol
3-[4-(4-Methoxyphenyl)-cyclohexenyl]-1,2-difluorbenzol
3-[4-(4-Ethoxyphenyl)-cyclohexenyl]-1,2-difluorbenzol
3-[4-(4-Propoxyphenyl)-cyclohexenyl]-1,2-difluorbenzol
3-[4-(4-Pentoxyphenyl)-cyclohexenyl]-1,2-difluorbenzol
3-[4-(4-Heptoxyphenyl)-cyclohexenyl]-1,2-difluorbenzol
1,4-Di-(4-propylcylohexenyl)-2,3-difluorbenzol

1,4-Di-(4-pentylcylohexenyl)-2,3-difluorbenzol
3-(4-Methylcyclohexenyl)-1,2-difluorbenzol
3-(4-Ethylcyclohexenyl)-1,2-difluorbenzol
3-(4-Propylcyclohexenyl)-1,2-difluorbenzol, $Kp_{0,5}$: 91 °C
3-(4-Pentylcyclohexenyl)-1,2-difkuorbenzol, $Kp_{0,5}$: 123 °C
3-(4-Heptylcyclohexenyl)-1,2-difluorbenzol
4-Methyl-1-(4-pentylcyclohexenyl)-2,3-difluorbenzol
4-Ethyl-1-(4-pentylcyclohexenyl)-2,3-difluorbenzol
4-Propyl-1-(4-pentylcyclohexenyl)-2,3-difluorbenzol
4-Pentyl-1-(4-pentylcyclohexenyl)-2,3-difluorbenzol
4-Heptyl-1-(4-pentylcyclohexenyl)-2,3-difluorbenzol
4-Methoxy-1-(4-pentylcyclohexenyl)-2,3-difluorbenzol
4-Ethoxy-1-(4-pentylcyclohexenyl)-2,3-difluorbenzol
4-Propoxy-1-(4-pentylcyclohexenyl)-2,3-difluorbenzol
4-Pentoxy-1-(4-pentylcyclohexenyl)-2,3-difluorbenzol
4-Heptoxy-1-(4-pentylcyclohexenyl)-2,3-difluorbenzol.

Beispiel 6

Darstellung von 3-Propenyl-1,2-difluorbenzol

Zu der nach 1b) hergestellten Lösung von 1,2-Difluorphenyllithium in THF wird eine Lösung von 0,13 mol Propionaldehyd in 30 ml THF getropft. Anschließend wird auf 0 °C erwärmt und wie üblich aufgearbeitet. Das entstandene Rohprodukt des Carbinols wird in 80 ml Toluol gelöst und mit 1 g p-TsOH 1 Stunde am Wasserabscheider zum Sieden erhitzt. Danach werden 0,2 ml konzentrierte Schwefelsäure zugesetzt und nochmals 1 Stunde, wie oben, erhitzt. Abschließend wird wie üblich aufgearbeitet, $Kp_{30}$: 105 °C.

Analog werden hergestellt:
3-Butenyl-1,2-difluorbenzol
3-Pentenyl-1,2-difluorbenzol
3-Hexenyl-1,2-difluorbenzol
3-Heptenyl-1,2-difluorbenzol
3-Octenyl-1,2-difluorbenzol.

Beispiel 7

Darstellung von 3-Propyl-1,2-difluorbenzol

In eine mit 5 g Pd/C suspendierte Lösung von 0,13 mol 3-Propenyl-1,2-difluorbenzol (erhältlich nach Beispiel 11) in 100 ml THF wird bei Raumtemperatur bis zur Sättigung Wasserstoff eingeleitet. Nach 3stündigem Rühren wird wie üblich aufgearbeitet, $Kp_{30}$: 72 °C.

Analog werden hergestellt:
3-Butyl-1,2-difluorbenzol
3-Pentyl-1,2-difluorbenzol
3-Hexyl-1,2-difluorbenzol
3-Heptyl-1,2-difluorbenzol
3-Octyl-1,2-difluorbenzol.

Beispiel 8

Darstellung von 3-(4-Pentylphenyl)-1,2-difluorbenzol

Eine Lösung von 0,225 mol 3-(4-Pentylcyclohexenyl)-1,2-difluorbenzol (analoges Beispiel 6) und 0,55 mol DDQ in 1,5 1 Toluol wird 2 Stunden zum Sieden erhitzt. Anschließend wird wie üblich aufgearbeitet, $Kp_{0,5}$: 125 °C.

Analog werden hergestellt:
3-(4-Methylphenyl)-1,2-difluorbenzol
3-(4-Ethylphenyl)-1,2-difluorbenzol

3-(4-Propylphenyl)-1,2-difluorbenzol
3-(4-Heptylphenyl)-1,2-difluorbenzol

Beispiel 9

Darstellung von 3-(4-Methylcyclohexyl)-1,2-difluorbenzol

3-(4-Methylcyclohexenyl)-1,2-difluorbenzol (analoges Beispiel 6) wird, wie in Beispiel 12 beschrieben, hydriert.

Analog werden hergestellt:
3-(4-Ethylcyclohexyl)-1,2-difluorbenzol
3-(4-Propylcyclohexyl)-1,2-difluorbenzol
3-(4-Pentylcyclohexyl)-1,2-difluorbenzol
3-(4-Heptylcyclohexyl)-1,2-difluorbenzol.

Beispiel 10

Darstellung von 4-Methyl-1-(5-methylpyridin-2-yl)-2,3-difluorbenzol

3-Methyl-1,2-difluorbenzol wird nach 1a) metalliert (10 mmol). Nach Zugabe von 10 mmol Chlortriisopropylorthotitanat wird auf -20 °C erwärmt und 15 Minuten weitergerührt. Danach wird mit 80 mg Tetrakis-(triphenylphosphin)palladium(0) und 10 mmol 2-Brom-5-methylpyridin versetzt. Abschließend wird noch 18 Stunden bei Raumtemperatur weitergerührt und wie üblich aufgearbeitet.

Analog werden hergestellt:
4-Ethyl-1-(5-methylpyridin-2-yl)-2,3-difluorbenzol
4-Propyl-1-(5-methylpyridin-2-yl)-2,3-difluorbenzol
4-Pentyl-1-(5-methylpyridin-2-yl)-2,3-difluorbenzol
4-Heptyl-1-(5-methylpyridin-2-yl)-2,3-difluorbenzol
4-Methoxy-1-(5-methylpyridin-2-yl)-2,3-difluorbenzol
4-Ethoxy-1-(5-methylpyridin-2-yl)-2,3-difluorbenzol, Fp.: 73 °C
4-Propoxy-1-(5-methylpyridin-2-yl)-2,3-difluorbenzol
4-Pentoxy-1-(5-methylpyridin-2-yl)-2,3-difluorbenzol
4-Heptoxy-1-(5-methylpyridin-2-yl)-2,3-difluorbenzol.

Verwendungsbeispiel

Darstellung von trans-4-Pentyl-cyclohexancarbonsäure 4-ethoxy-2,3-difluorphenolat

10 mmol trans-4-Pentyl-cyclohexancarbonsäurechlorid und 10 mmol 4-Ethoxy-2,3-difluorphenol werden in 20 ml Dichlormethan gelöst. Nach Zugabe von 15 mmol Pyridin wird 6 Stunden bei Raumtemperatur gerührt und wie üblich aufgearbeitet (Umkistallisation aus Methanol), K 48 N 62.5 I.

Analog werden hergestellt:
trans-4-Pentyl-cyclohexancarbonsäure 4-methoxy-2,3-difluorphenolat
trans-4-Pentyl-cyclohexancarbonsäure 4-propoxy-2,3-difluorphenolat
trans-4-Pentyl-cyclohexancarbonsäure 4-pentoxy-2,3-difluorphenolat
trans-4-Pentyl-cyclohexancarbonsäure 4-heptoxy-2,3-difluorphenolat
trans-4-Pentyl-cyclohexancarbonsäure 4-octoxy-2,3-difluorphenolat
trans-4-Pentyl-cyclohexancarbonsäure 4-methyl-2,3-difluorphenolat
trans-4-Pentyl-cyclohexancarbonsäure 4-ethyl-2,3-difluorphenolat
trans-4-Pentyl-cyclohexancarbonsäure 4-propyl-2,3-difluorphenolat
trans-4-Pentyl-cyclohexancarbonsäure 4-pentyl-2,3-difluorphenolat
trans-4-Pentyl-cyclohexancarbonsäure 4-heptyl-2,3-difluorphenolat.

Beispiel 11

Darstellung von 4'-Heptyloxy-2,3-difluor-biphenyl-4-carbonsäure

0,1 mol 2,3-Difluor-4'-heptyloxybiphenyl wird nach 1b) metalliert. Man rührt 3 Stunden bei -78 °C und kippt dann das Reaktionsgemisch in einem Schwung auf 200 g gestoßenes Trockeneis. Anschließend wird wie üblich aufgearbeitet.

Beispiel 12

Darstellung von 4-Hydroxy-2,3-difluor-4'-heptyloxybiphenyl

0,1 mol 2,3-Difluor-4'-heptyloxybiphenyl (hergestellt durch Metallierung von 2,3-Difluorbenzol nach 1b) und Umsetzung mit Heptyloxycyclohexanon sowie anschließende Dehydratisierung mit Toluol/pTSOH am Wasserabscheider (analog Beispiel 6) und Aromatisierung mit DDQ (analog Beispiel 13)) wird nach 1b) metalliert (3 Stunden Rühren bei -78 °C). In der Zwischenzeit gibt man zu einer Lösung von 0,12 mol tert.-Butylhydroperoxid in 50 ml Ether innerhalb von 30 Minuten 70 ml einer 2n Lösung von Ethylmagnesiumbromid in Ether zu. Die so bereitete Lösung tropft man vorsichtig zu der auf -78 °C gekühlten Lösung des metallierten 2,3-Difluor-4'-heptyloxybiphenyls, läßt dann auf Raumtemperatur erwärmen und rührt dann nochmals 2 Stunden. Anschließend wird wie üblich aufgearbeitet.

Beispiel 13

Darstellung von 4-Ethoxy-2,3-difluorphenol

0,02 mol 2,3-Difluorethoxybenzol werden nach 1b metalliert. Anschließend werden 0,02 mol Trimethylborat bei einer Temperatur von etwa -55° bis -60 °C während 0,5 h zugetropft. Die entstandene Suspension wird noch 0,5 h weitergerührt, wobei die Temperatur auf etwa -20° ansteigt. Man gibt 1,5 ml 98%ige Essigsäure hinzu. Nach etwa 15 min wird auf etwa -35° abgekühlt und eine Lösung von 30%iger $H_2O_2/H_2O$ (5 ml/2 ml) hinzugetropft. Nach 2 h Rühren unter langsamem Erwärmen auf Raumtemperatur wird die organische Phase separiert und die wäßrige mit Methyl-tert.butylether extrahiert. Abschließend wird mit gesättigter NaCl-Lösung gewaschen, getrocknet und eingedampft.

Analog werden hergestellt:

4-Methyl-2,3-difluorphenol
4-Ethyl-2,3-difluorphenol
4-Propyl-2,3-difluorphenol
4-Butyl-2,3-difluorphenol
4-Pentyl-2,3-difluorphenol, $Kp_{0.5}$: 77 °C
4-Hexyl-2,3-difluorphenol
4-Octyl-2,3-difluorphenol
4-Nonyl-2,3-difluorphenol
4-Decyl-2,3-difluorphenol
4-Heptyl-2,3-difluorphenol
4-Methoxy-2,3-difluorphenol
4-Propoxy-2,3-difluorphenol
4-Butoxy-2,3-difluorphenol
4-Pentoxy-2,3-difluorphenol
4-Hexoxy-2,3-difluorphenol
4-Heptoxy-2,3-difluorphenol
4-Octoxy-2,3-difluorphenol
4-Nonoxy-2,3-difluorphenol
4-Decoxy-2,3-difluorphenol
4-(4-Methylphenyl)-2,3-difluorphenol
4-(4-Ethylphenyl)-2,3-difluorphenol
4-(4-Propylphenyl)-2,3-difluorphenol
4-(4-Pentylphenyl)-2,3-difluorphenol
4-(4-Heptylphenyl)-2,3-difluorphenol
4-(4-Methoxyphenyl)-2,3-difluorphenol

4-(4-Propoxyphenyl)-2,3-difluorphenol
4-(4-Pentoxyphenyl)-2,3-difluorphenol.

Beispiel 14

Darstellung von 4-Octyloxy-1-[2-(trans-4-pentylcyclohexyl)-oxoethyl]-2,3-difluorbenzol

0,03 mol 3-Octyloxy-1,2-difluorbenzol werden nach 1a) metalliert. Anschließend wird eine Suspension von 0,03 mol Zinkbromid in 20 ml THF langsam zugegeben und das Gemisch auf -10 °C erwärmt. Danach werden 50 mg $Ni(Ph_3P)_2Cl_2$ und eine Lösung von 0,03 mol 2-(trans-4-Pentylcyclohexyl)-essigsäurechlorid in 20 ml THF zugegeben. Das Gemisch wird auf Raumtemperatur erwärmt, wie üblich aufgearbeitet und aus Methanol/Ethanol (1:1) umkristallisiert, Fp: 59 °C, K 59 $S_A$ (55) I.

**Patentansprüche**

1. Verbindungen der Formel I

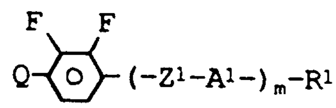

worin
Q

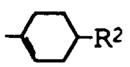

oder $R^3$

$R^2$ H oder einen Alkyl- oder Alkenylrest mit jeweils 1 bis 15 C-Atomen, worin eine oder mehrere nicht benachbarte $CH_2$-Gruppen durch O-Atome ersetzt sein können,
$R^3$ M, $-OR^4$, $-CO-OR^4$ oder $-O-COR^4$,
M Li, Na oder K,
$R^4$ H oder einen Alkylrest mit 1 bis 12 C-Atomen,
$Z^1$ $-OCH_2-$, $-CH_2O-$, $-CH_2CH_2-$, -CH = CH-, -C≡C- oder eine Einfachbindung,
$A^1$ jeweils unabhängig voneinander einen
a) 1,4-Cyclohexylenrest, worin eine oder zwei nicht benachbarte $CH_2$-Gruppen durch O-Atome ersetzt sein können,
b) 1,4-Phenylenrest, worin eine oder mehrere CH-Gruppen durch N ersetzt sein können,
c) Rest aus der Gruppe 1,4-Cyclohexenylen, Piperin-1,4-diyl, Bicyclo(2,2,2)octylen, Naphthalin-2,6-diyl oder 1,2,3,4-Decahydronaphthalin-2,6-diyl oder 1,2,3,4-Tetrahydronaphtalin,
wobei der Rest b) ein- oder zweifach durch F, Cl und/oder $-CH_3$ substituiert sein kann,
m 0, 1, 2 oder 3,
$R^1$ einen Alkyl-, Perfluoralkyl- oder Alkenylrest mit jeweils 1-15 C-Atomen, worin eine oder mehrere nicht benachbarte $CH_2$- bzw. $CF_2$-Gruppen durch O- und/oder S-Atome ersetzt sein können,
$R^1$ in dem Fall
m gleich 0 auch F, Cl oder Br
sein kann, mit der Maßgabe, daß Q und $R^1$ nicht gleichzeitig Reste, ausgewählt aus der Gruppe F, Cl, Br, $CF_3$ und -OH sein dürfen, bedeuten.
wobei in Verbindungen, in denen alle Reste $A^1$, ausgewählt aus der Gruppe unsubstituiertes oder ein- oder zweifach durch F substituiertes 1,4-Phenylen, bedeuten, mindestens ein $Z^1$ $-OCH_2-$, $-CH_2O-$, $-CH_2CH_2-$, -CH = CH- oder -C≡C- bedeutet,
sowie deren reaktionsfähige Derivate.

2. Verbindungen nach Anspruch 1, dadurch gekennzeichnet, daß M Li oder K ist.

**3.** Verbindungen der Formeln Ia1, Ia5 und Ia7,

M-$A^0$-$R^1$ Ia1

HO-$A^0$-$R^1$ Ia5

HO-OC-$A^0$-$R^1$ Ia7

worin

$A^0$

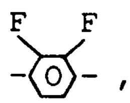

M Li oder K, und

$R^1$ einen Alkyl-, Alkenyl- oder Alkoxyrest mit bis zu 12 C-Atomen bedeutet.

**4.** Verfahren zur Herstellung von Verbindungen der Formel I, dadurch gekennzeichnet, daß man Verbindungen, die ansonsten der Formel I entsprechen, worin aber Q H bedeutet, mit einem metallorganischen Reagens in der 4-Position deprotoniert und gegebenenfalls weiter mit einem Elektrophil, wie Halogen (gegebenenfalls mit anschließender Substitution des Halogenatoms gegen -CN), Ethylenoxid, einem Peroxid, Disulfid oder Schwefel (gegebenenfalls mit anschließender Veresterung), einer reaktiven Carbonylverbindung oder Kohlendioxid (gegebenenfalls mit anschließender Veresterung oder Dehydratisierung), umsetzt.

**5.** Verfahren nach Anspruch 4, dadurch gekennzeichnet, daß die Reaktion in inerten Lösungsmitteln bei tiefen Temperaturen unter Schutzgas durchgeführt wird.

**6.** Verbindungen der Formel II,

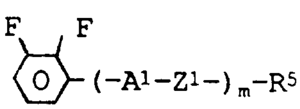

II

worin

$A^1$, $Z^1$ und m die in Anspruch 1 angegebene Bedeutung haben und

$R^5$ im Falle m ungleich 0
H oder einen Alkyl-, Perfluoralkyl- oder Alkenylrest mit jeweils 1 bis 15 C-Atomen bedeutet, wobei in diesen Resten eine oder mehrere nicht benachbarte $CH_2$- bzw. $CF_2$-Gruppen durch O- und/oder S-Atome ersetzt sein können, ausgenommen H für den Fall, daß $-(-A^1-Z^1-)_m-$ einen unsubstituierten oder einen einfach durch F substituierten 1,4-Phenylenrest bedeutet,
im Falle m gleich 0
einen unverzweigten Alkylrest mit 2 bis 15 C-Atomen oder einen Perfluor- oder Alkenylrest mit 2 bis 15 C-Atomen, wobei in diesen Resten eine oder mehrere nicht benachbarte $CH_2$- bzw. $CF_2$-Gruppen durch O- und/oder S-Atome ersetzt sein können.

**7.** Verbindungen der Formeln,

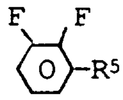

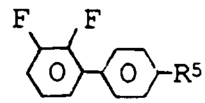

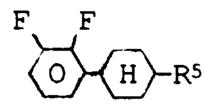

worin $R^5$ die in Anspruch 6 angegebene Bedeutung hat.

**8.** Verwendung von Verbindungen der Formel I als Zwischenprodukte zur Synthese von flüssigkristallinen Verbindungen.

## Claims

**1.** Compounds of the formula I

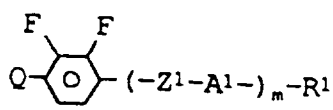

in which
Q is

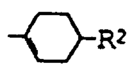

or $R^3$
$R^2$ is H or an alkyl or alkenyl radical in each case having 1 to 15 C atoms, in which one or more non-adjacent $CH_2$ groups can be replaced by O atoms,
$R^3$ is M, $-OR^4$, $-CO\text{-}0R^4$ or $-O\text{-}COR^4$,
M is Li, Na or K,
$R^4$ is H or an alkyl radical having 1 to 12 C atoms,
$Z^1$ is $-OCH_2-$, $-CH_2O-$, $-CH_2CH_2-$, $-CH=CH-$, $-C\equiv C-$ or a single bond,
$A^1$ in each case independently of one another are a

a) 1,4-cyclohexylene radical, in which one or two non-adjacent $CH_2$ groups can be replaced by O atoms,
b) 1,4-phenylene radical, in which one or more CH groups can be replaced by N,
c) radical from the group consisting of 1,4-cyclohexenylene, piperidine-1,4-diyl, bicyclo[2.2.2]-octylene, naphthalene-2,6-diyl or 1,2,3,4-decahydronaphthalene-2,6-diyl or 1,2,3,4-tetrahydronaphthalene, where the radical b) can be mono- or disubstituted by F, Cl and/or $-CH_3$,

m is 0, 1, 2 or 3,
$R^1$ is an alkyl, perfluoroalkyl or alkenyl radical in each case having 1-15 C atoms, in which one or more

non-adjacent $CH_2$ or $CF_2$ groups can be replaced by O and/or S atoms,
$R^1$, if m is equal to 0,
can also be F, Cl or Br, with the proviso that Q and $R^1$ must not simultaneously be radicals selected from the group consisting of F, Cl, Br, $CF_3$ and -OH,
where, in compounds in which all radicals $A^1$ are selected from the group consisting of 1,4-phenylene which is unsubstituted or mono- or disubstituted by F, at least one $Z^1$ is $-OCH_2-$, $-CH_2O-$, $-CH_2CH_2-$, -CH = CH- or -C≡C-, and their reactive derivatives.

**2.** Compounds according to Claim 1, characterized in that M is Li or K.

**3.** Compounds of the formulae Ia1, Ia5 and Ia7,

$M-A^0-R^1$ Ia1

$HO-A^0-R^1$ Ia5

$HO-OC-A^0-R^1$ Ia7

in which
$A^0$ is

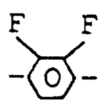 ,

M is Li or K, and
$R^1$ is an alkyl, alkenyl or alkoxy radical having up to 12 C atoms.

**4.** Process for the preparation of compounds of the formula I, characterized in that compounds which otherwise correspond to the formula I but in which Q is H are deprotonated in the 4-position using an organometallic reagent and are optionally further reacted with an electrophile, such as halogen (optionally with subsequent substitution of the halogen atom by -CN), ethylene oxide, a peroxide, disulfide or sulfur (optionally with subsequent esterification), a reactive carbonyl compound or carbon dioxide (optionally with subsequent esterification or dehydration).

**5.** Process according to Claim 4, characterized in that the reaction is carried out in inert solvents at low temperatures under protective gas.

**6.** Compounds of the formula II

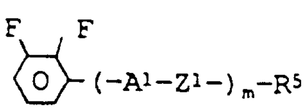 II

in which
$A^1$, $Z^1$ and m have the meaning indicated in Claim 1 and
$R^5$, if m is not equal to 0,
is H or an alkyl radical, perfluoroalkyl or alkenyl radical in each case having 1 to 15 C atoms, where in these radicals one or more non-adjacent $CH_2$ or $CF_2$ groups can be replaced by O and/or S atoms, excluding H in the case in which $-(-A^1-Z^1-)_m-$ is a 1,4-phenylene radical which is unsubstituted or monosubstituted by F,
if m is equal to 0
is an unbranched alkyl radical having 2 to 15 C atoms or a perfluoro or alkenyl radical having 2 to 15 C atoms, where in these radicals one or more non-adjacent $CH_2$ or $CF_2$ groups can be replaced by O and/or S atoms.

**7.** Compounds of the formulae

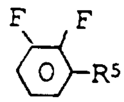

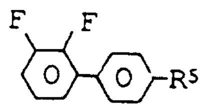

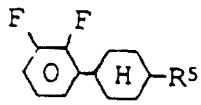

in which $R^5$ has the meaning indicated in Claim 6.

**8.** Use of compounds of the formula I as intermediates for the synthesis of liquid crystalline compounds.

**Revendications**

**1.** Composés de formule I

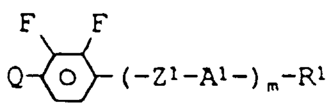

dans laquelle

Q représente

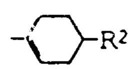

ou $R^3$

$R^2$ représente H ou un radical alkyle ou alcényle avec à chaque fois de 1 à 15 atomes de carbone, où un ou plusieurs groupes $CH_2$ non voisins peuvent être remplacés par des atomes d'oxygène,

$R^3$ représente M, $-OR^4$ , $-CO-OR^4$ ou $-O-COR^4$ ,

M représente Li, Na ou K,

$R^4$ représente H ou un radical alkyle en $C_1$ à $C_{12}$ ,

$Z^1$ représente $-OCH_2-$ , $-CH_2O-$ , $-CH_2CH_2-$ , $-CH=CH-$ , $-C\equiv C-$ ou une liaison simple,

$A^1$ représente à chaque fois indépendamment

a) un radical 1,4-cyclohexylène dans lequel un ou deux groupes $CH_2$ peuvent être remplacés par des atomes d'oxygène,

b) un radical 1,4-phénylène dans lequel un ou plusieurs groupes CH peuvent être remplacés par N,

c) un radical du groupe 1,4-cyclohexénylène, pipéridine-1,4-diyle, bicyclo(2,2,2)octylène, naphtalène-2,6-diyle ou 1,2,3,4-décahydronaphtalène-2,6-diyle, ou 1,2,3,4-tétrahydronaphtalène, où le radical b) peut être mono- ou disubstitué par F, Cl et/ou $-CH_3$ ,

m vaut 0, 1, 2 ou 3,

$R^1$ représente un radical alkyle, perfluoroalkyle ou alcényle avec à chaque fois de 1 à 15 atomes de carbone, où un ou plusieurs groupes $CH_2$ ou $CF_2$ non voisins peuvent être remplacés par des atomes d'oxygène et/ou de soufre,

$R^1$ dans le cas où m vaut 0 représente également F, Cl ou Br, sous réserve que Q et $R^1$ ne représentent pas simultanément des radicaux choisis dans le groupe F, Cl, Br, $CF_3$ et -OH; où dans les composés dans lesquels tous les radicaux $A^1$ sont choisis dans le groupe constitué par un 1,4-phénylène non substitué ou mono- ou disubstitué par F, au moins un radical $Z^1$ représente $-OCH_2-$, $-CH_2O-$ , $-CH_2CH_2-$ , -CH=CH- ou -C≡C- ,

ainsi que leurs dérivés réactifs.

**2.** Composés selon la revendication 1, caractérisés en ce que M représente Li ou K.

**3.** Composés de formules Ia1, Ia5 et Ia7 ,

$M-A^0-R^1$ Ia1

$HO-A^0-R^1$ Ia5

$HO-OC-A^0-R^1$ Ia7

dans lesquelles

$A^0$ représente

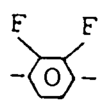 ,

M représente Li ou K, et

$R^1$ représente un radical alkyle, alcényle ou alcoxy ayant jusqu'à 12 atomes de carbone.

**4.** Procédé de préparation de composés de formule I, caractérisé en ce qu'on fait réagir des composés qui sinon correspondent à la formule I , mais où Q représente H, avec un réactif organo-métallique déprotoné en position 4, et le cas échéant

plus avant avec un agent électrophile, comme un halogène (ceci étant suivi le cas échéant par une substitution de l'atome d'halogène contre -CN), l'oxyde d'éthylène, un peroxyde, un disulfure ou le soufre (ceci étant le cas échéant suivi par une estérification), un composé carbonyle réactif ou le dioxyde de carbone (ceci étant le cas échéant suivi par une estérification ou une déshydratation).

**5.** Procédé selon la revendication 4, caractérisé en ce qu'on conduit la réaction dans des solvants inertes à basse température sous un gaz protecteur.

**6.** Composés de formule II ,

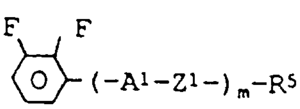

II

dans laquelle

$A^1$, $Z^1$ et m ont la signification donnée dans la revendication 1 et

$R^5$ dans le cas où m est différent de zéro représente H ou un radical alkyle, perfluoroalkyle ou alcényle avec à chaque fois de 1 à 15 atomes de carbone, où dans ces radicaux un ou plusieurs groupes $CH_2$ ou $CF_2$ non voisins peuvent être remplacés par des atomes d'oxygène et/ou de soufre, à l'exception de H dans le cas où $-(-A^1-Z^1-)_m-$ représente un radical 1,4-phénylène non substitué ou mono-substitué par F, dans le cas où m est égal à zero représente un radical alkyle non ramifié en $C_2$ à $C_{15}$ ou un radical perfluoro ou alcényle en $C_2$ à $C_{15}$ , où dans ces radicaux un ou plusieurs groupes $CH_2$ ou $CF_2$ non voisins peuvent être remplacés par des atomes d'oxygène et/ou de soufre.

**7.** Composés de formules

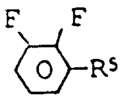

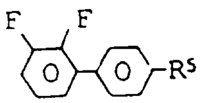

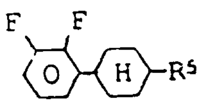

dans lesquelles $R^5$ a la signification donnée dans la revendication 6.

**8.** Application de composés de formule I comme produits intermédiaires de la synthèse de composés à cristaux liquides.